# EUROPÄISCHE PATENTSCHRIFT

(11) **EP 1 614 440 B1**
(45) Veröffentlichungstag und Bekanntmachung des Hinweises auf die Patenterteilung: **13.02.2008**
(21) Anmeldenummer: 05405414.3
(22) Anmeldetag: 28.06.2005
(51) Int. Cl.: A61M 5/32

(54) **Verfahren zum Vernichten von chirurgischen Instrumenten durch Schmelzen und Vorrichtung zum Ausüben des Verfahrens**
Method of destroying surgical instruments by melting and apparatus for carrying out the method
Méthode de destruction d'instuments chirurgicaux par fusion et dispositif pour réaliser cette méthode

(30) Priorität: 08.07.2004 CH 11462004
(43) Veröffentlichungstag der Anmeldung: 11.01.2006
(73) Patentinhaber: Banner Ventures LLC, Morristown NJ 07962 (US)
(72) Erfinder: Veschi, Raffaele, 01408-020 Sao Paolo (BR)
(74) Vertreter: Gaggini, Carlo

(56) Entgegenhaltungen:
- US-A- 5 441 622
- US-A1- 2002 144 896

## Beschreibung

Die vorliegende Erfindung betrifft ein Verfahren zum Vernichten von chirurgischen Instrumenten durch Schmelzen, gemäss dem Oberbegriff des Anspruchs 1, und eine Vorrichtung zum Ausüben des Verfahrens gemäss dem Oberbegriff des Anspruchs 4.

Die Vernichtung gebrauchter kleiner chirurgischer Instrumente jeder Art durch Schmelzen, insbesondere von einmal zu verwendenden Injektionsnadeln von Spritzen sowie von Messerchen und Skalpellen, hat grosse Bedeutung erlangt in Spitälern, Zahnarztpraxen, und generell überall, wo Pflegepersonal, in erster Linie Ärzte und Krankenschwestern, mit den Patienten in physischen Kontakt treten müssen. In der Tat ist die Gefahr, von gefährlichen Viren und Bakterien schwerster Krankheiten wie HIV oder bestimmter Hepatitis-Arten angesteckt zu werden, für das Sanitätspersonal sehr hoch, das deshalb alle möglichen Schutzmassnahmen vorkehren muss. Dabei spielen jene eine fundamentale Rolle, die das rasche und vollständige Entfernen der infizierten Instrumente ohne Zwischentransporte betreffen, wobei es nicht genügt, sie in dichten Behältern zu entfernen, da deren Handhabung eine mögliche Gefahrenquelle darstellt. Insbesondere sind es die versehentlichen Stichverletzungen bei der Verwendung von Spritzen bei therapeutischen Behandlungen, welche die damit arbeitenden Personen am meisten beschäftigen, sowie auch die Behörden, die genaue Regeln zum Schutz vor biologischen Agenzien erlassen haben, und die das Konzept des "Expositions-Risikos" geschaffen haben.

In den letzten Jahren sind zahlreiche Vorschläge gemacht worden zur Ausschaltung der genannten Risiken, wodurch auch eine umfangreiche Patentliteratur darüber entstanden ist sowie einige Ansätze zur praktischen Realisierung.

Diese Lösungen lassen sich in zwei grosse Kategorien einteilen: Jene des mechanischen Vernichtens mittels Abschleifen oder Zerbrechens das Objektes in kleinste Teile, die zu entfernen sind (eine Lösung, die jedoch die Bedingungen der sicheren und vollständigen Entfernung der pathogenen Keime nicht voll erfüllt, und die hier nur vollständigkeithalber erwähnt ist), und jene, welche ein Schmelzen der Objekte mittels thermischer Behandlung vorsehen. Diese Lösung ist offensichtlich besser als jene der mechanischen Zerstörung, wobei sich die vorliegende Erfindung ausschliesslich auf die Vernichtung durch Schmelzen konzentriert.

Vollständigkeitshalber seien hier jedoch auch zwei bekannte Beispiele von Vorrichtungen für die Vernichtung genannt, die sich auf mechanische Behandlung stützen: Dabei handelt es sich beispielsweise um Vorrichtungen, wie sie in der US-5'979'275 und in der WO03/018090A1 beschrieben sind.

Bezüglich der Vernichtung durch Schmelzen hingegen besteht nur die Qual der Wahl unter einer enormen Vielzahl von Dokumenten, die jedoch alle durch einen gemeinsamen Aspekt gekennzeichnet sind, nämlich dass bei allen bekannten Lösungen die Verbrennung bzw. das Schmelzen mittels eines elektromagnetischen Feldes bewerkstelligt wird, das zwischen zwei Elektroden bei hohem Spannungsunterschied angelegt wird.

Von diesen Vorschlägen verdient beispielsweise die EP-0476823B1 Erwähnung, die mit elektromagnetischen Mikrowellen arbeitet, welche eine PlasmaEntladung erzeugen, oder die WO-94/13346, wo zwei Elektroden vorgesehen sind, von denen eine rotiert, um eine Verunreinigung der Elektroden zu vermeiden, wobei die WO-97126035 in den Zielsetzungen und auch in der Auslegung dem genannten Dokument sehr ähnlich ist, ebenso wie die FR-2745187 und die FR-2748185.

Die WO-97/33639, die für diese Art von Apparaten typisch ist, versucht gegen einen allen bekannten Vorrichtungen zum Schmelzen mittels elektrischer Entladung zwischen zwei Elektroden innewohnenden Nachteil Abhilfe zu schaffen, nämlich gegen die Verschmutzung der Elektroden durch die vom Schmelzen herrührenden Schlacken. Solche Verschmutzung kann infolge der Ablagerung von Schlacke ein Versagen des Betriebs der Apparatur hervorrufen, das sehr gefährlich sein kann, weil die Zuverlässigkeit eines solchen Apparates zur Vernichtung von Spritzen und Ähnlichem absolut sicher sein muss. Auch nur ein einziger Fall von Nicht-Funktionieren oder von bloss teilweisem Funktionieren kann die körperliche Unversehrtheit der Bedienungsperson ernsthaft in Gefahr bringen, die sich absolut auf den Apparat verlassen können muss. Die Notwendigkeit, die auf der einen oder der anderen der beiden Elektroden abgelagerten Schlacken entfernen zu müssen, stellt daher einen der Hauptnachteile der bekannten Technik dar.

Ein weiteres interessantes Dokument, das die Grenzen der bekannten Technik klar aufzeigt, ist US-6'649'857 neuesten Datums (erteilt 2003) in welchem die spezielle Lösung beansprucht wird, dass ein ringförmiger Raum um die Spritze vorgesehen ist, wobei dieser Raum zwischen den beiden herkömmlichen Elektroden geschaffen wird. Dieses Dokument zeigt also die grosse Bedeutung der Formanpassung der Elektroden an das zu zerstörende Objekt, was eine hinderliche Begrenzung der allgemeinen Anwendbarkeit des Apparates darstellt. Auch bezüglich dieses Aspektes bietet die Lösung mit den beiden Elektroden nicht das, was die Praxis erwartet, nämlich Zuverlässigkeit, vielseitige Anwendbarkeit und geringes Gewicht der Vorrichtung.

Dies sind die Zielsetzungen der vorliegenden Erfindung, die sich hauptsächlich darauf ausrichten, die genannten Nachteile des Standes der Technik auszuschalten, die mit der Verwendung von zwei Elektroden zur Erzeugung eines Feldes elektromagnetischer Wellen mit entsprechender Bildung elektrischer Entladungen einhergehen.

Diese Zielsetzungen werden erreicht dank einem Verfahren mit den Eigenschaften gemäss dem kennzeichnenden Teil des Anspruchs 1. Die Neuheit der vorliegenden Erfindung besteht dabei in der Verwendung einer Flamme von höchster Temperatur (wobei mit dieser Bezeichnung eine Temperatur von einigen tausend Grad Celsius gemeint ist, die auf jeden Fall im Stande ist, jedes beliebige Metall zu schmelzen, aus dem das zu vernichtende Instrument hergestellt sein kann), um das Schmelzen der Teile des Instrumentes, die vernichtet werden sollen, zu bewerkstelligen, die mittels Verbrennung eines Gemischces von Wasserstoff und Sauerstoff erzeugt wird.

Die vorliegende Erfindung zielt also darauf ab, sich eine Technologie zu Nutzen zu machen, nämlich die das Erzeugen einer Flamme sehr hoher Temperatur unter Verwendung der bekannten Generatoren für Wasserstoff und Sauerstoff, die auf der elektrolytischen Dissoziation von Wasser beruht, die in der Praxis häufig angewendet wird und bis heute insbesondere zum Hartlöten bei Goldschmiedearbeiten, bei der Schmuckherstellung, in der Zahntechnik, in der Elektromechanik und der Elektronik, usw., verwendet wird. Für diese Technologie steht heute eine vollständige Reihe von Apparaten zur Verfügung, deren Leistung von 20 kW und mehr bis zu 280 W reicht, mittels welchen Mengen von über 3500 l/h Wasserstoff bis weniger als 40 l/h herstellen lassen: Dabei handelt es sich um kleinformatige Apparate, besonders für kleinere Leistungen, die in der Handhabung und im Gebrauch einfach sind, und die sich für den Anwendungsbereich der vorliegenden Erfindung ideal eignen, wofür eine Flamme hoher Temperatur, aber kleine Abmessungen des Apparates erforderlich sind, wie auch die Abmessungen einer Injektionsnadel oder auch der Klingen kleiner Messerchen für Präzisionsoperationen klein sind. Apparate solcher Art werden beispielsweise von der Firma Elettronica Todescati s.r.l., Via Al Volta, 9 A/C - 36057 Arcugnano (VI) Italien, hergestellt und verkauft und sind in einem Katalog der verfügbaren Modelle unter dem Titel "Wasserstoff- und Sauerstoff-Generatoren" beschrieben, auf welchen sich hier alles bezieht, was die Erzeugung einer Flamme hoher Temperatur (>3000°C gemäss einer bevorzugten Realisierungsform der vorliegenden Erfindung) betrifft, mittels der Verbrennung von Wasserstoff und Sauerstoff, die beide mittels Elektrolyse durch Dissoziation von Wasser gewonnen werden. Diese Technologie kann daher als bekannt vorausgesetzt werden, weshalb hier nicht länger darauf eingegangen wird. Die vorliegende Erfindung besteht in einer neuartigen Anwendung der Möglichkeiten, welche diese an sich bekannte Technologie bietet.

In einer bevorzugten Realisierungsform der vorliegenden Erfindung ist sodann vorgesehen, dass das Schmelzen von einem Punkt ausgehend bis zu einem Endpunkt fortschreitet, wobei das Instrument auf einer Strecke zwischen diesen beiden Punkten fortschreitend gegen die Flamme hin vorgeschoben wird. Diese Lösung eignet sich besonders gut, wenn es darum geht, ein längliches Objekt durch Schmelzen zu vernichten, wie beispielsweise eine Injektionsnadel oder ein Messerchen, mit Hilfe einer klein dimensionierten und im Raum feststehenden Flamme.

Die Vorrichtung nach Anspruch 4 zum Ausüben des erfindungsgemässen Verfahrens ist dadurch gekennzeichnet, dass ein Schmelztiegel vorhanden ist, der mit den erforderlichen Öffnungen für die Eingabe und für den Rauchabzug versehen ist sowie mit einem Speiseröhrchen für das aus Wasserstoff und Sauerstoff gemischte Gas, das in einem Generator erzeugt wurde, der auf der katalytischen Dissoziation von Wasser basiert.

In einer bevorzugten Realisierungsform der vorliegenden Erfindung ist sodann vorgesehen, dass zur Verbesserung der leichten Bedienbarkeit und Transportierbarkeit des Apparates der Schmelztiegel in den Wasserstoff- und Sauerstoffgenerator integriert ist.

Zur Erleichterung des fortschreitenden Schmelzens des Instrumentes von einem Anfangspunkt bis zu einem Endpunkt, beispielsweise zwischen der Spitze und der Halterung der Injektionsnadel, ist in einer weiteren bevorzugten Realisierungsform der vorliegenden Erfindung vorgesehen, dass der Apparat mit einer Führungsvorrichtung ausgerüstet ist, mittels welcher das zu schmelzende Instrument in seiner Vorschubbewegung geführt wird, in der Regel gradlinig von einem Anfangspunkt bis zum Endpunkt des Schmelzvorgangs.

Weitere Ausführungsvarianten werden im folgenden anhand einiger praktischer Beispiele von Ausführungsformen der vorliegenden Erfindung unter Bezugnahme auf die begleitenden Abbildungen näher beschrieben. Darin zeigen die:
- Fig. 1: Die erfindungsgemässe Vorrichtung mit all ihren hauptsächlichen Elementen während der Vernichtung einer Injektionsnadel in schematischer Darstellung,
- Fig. 2: Die erfindungsgemässe Vorrichtung, wobei der Schmelztiegel direkt in den Generator eingebaut ist,
- Fig. 3: Eine vergrösserte Teilansicht der Einschiebe-Offnung mit einer Führungsvorrichtung zum Führen des zu vernichtenden Instrumentes während des Schmelzprozesses.

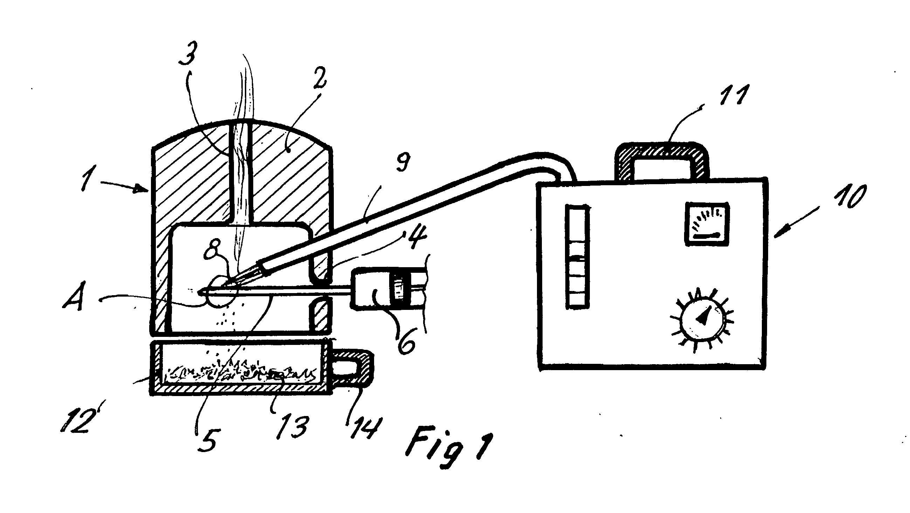

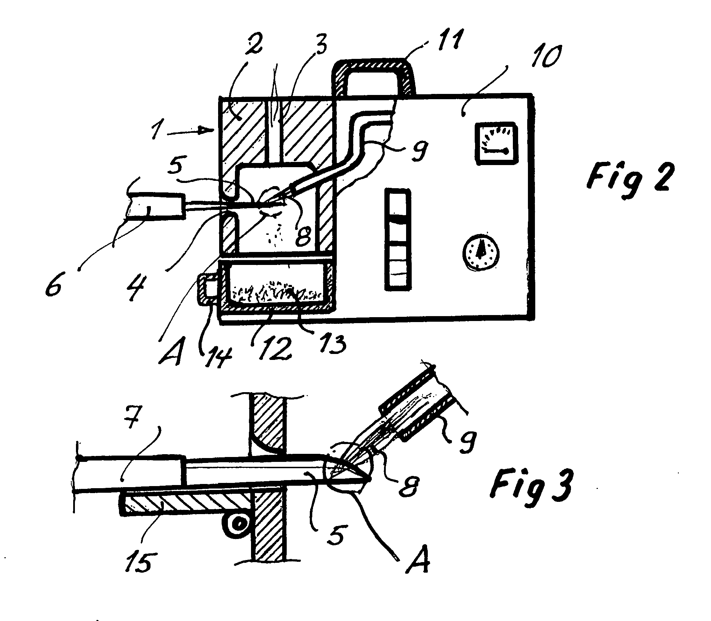

In der Fig. 1 ist der Schmelztiegel mit 1 bezeichnet, dessen Haube 2 mit einer Rauchabzugsöffnung 3 versehen ist. Der Schmelztiegel 1 ist vorzugsweise aus feuerfestem Material oder jedenfalls gut wärmeisolierendem Material gefertigt. Der Schmelztiegel 1 weist ferner eine Öffnung 4 auf, durch welche das zu schmelzende Instrument 5 eingeführt wird, das in den Figuren 1 und 2 als Injektionsnadel dargestellt (und in den Figuren 1 und 2 schematisch gezeigt ist) oder als Klinge eines Messerchens 7. In den Schmelztiegel 1 ragt auch ein Speiseröhrchen 9 für das Gasgemisch von Wasserstoff und Sauerstoff (Knallgas) bis an die geeignete Stelle, wo eine Flamme 8 am vorbestimmten Punkt des Einschmelzens des Instrumentes 5 erzeugt wird. Das Speiseröhrchen 9 ist erfindungsgemäss auf den Punkt ausgerichtet, an welchem das Instrument 5 im Innern des Tiegels 1 geschmolzen wird, genauer gesagt in solcher Weise, dass die heisseste Zone der Flamme, die gemäss einer bevorzugten Ausführungsform der vorliegenden Erfindung Temperaturen von über 3000°C erreicht, in direkten Kontakt mit dem zu schmelzenden Bereich des Instrumentes 5 (Injektionsnadel, Klinge, usw.) kommt, der dadurch sofort vernichtet und in Rauch und Schlacke aufgeht.

Zum Erzeugen einer Flamme von derartiger thermischer Leistung ist die Vorrichtung mit einem Wasserstoff- und Sauerstoffgenerator 10 ausgerüstet, der auf elektrolytische Dissoziation von Wasser basiert und durch das Speiseröhrchen 9 das Gasgemisch von Wasserstoff und Sauerstoff einspeist, das beim Verbrennen eine Flamme 8 von höchster Temperatur ergibt.

Der Wasserstoff- und Sauerstoffgenerator 10, der wie in der Einleitung erwähnt und beispielsweise im erwähnten Prospekt der Firma Elettronica Todescati s.r.l. beschrieben wird, ist selbstverständlich mit allen für den vorgesehenen Zweck erforderlichen Kontrollinstrumenten (Gastemperaturen, Druck, usw.) ausgerüstet, und ist auch mit einer mittels Mikroprozessor betriebenen Steuerung versehen, welche die Betriebssicherheit der Vorrichtung sicherstellt.

Femer ist festzuhalten, dass die Verbrennung von Wasserstoff und Sauerstoff keine schädlichen Abgase erzeugt, sondern lediglich Wasserdampf, wobei allenfalls aus dem Rauchabzug 3 austretender Rauch vom Schmelzen des Metalls herrührt, aus dem das zu vernichtende Instrument 5 gefertigt ist.

In der allgemeinsten Ausführungsform gemäss der Fig. 1 sind der Schmelztiegel 1 und der Wasserstoff- und Sauerstoffgenerator 10 als separate Elemente ausgebildet, die durch das Speiseröhrchen 9 miteinander verbunden sind, das in der Regel ein flexibler Gummischlauch oder ähnlichem Material ist. Der Generator 10 ist mit einem Handgriff 11 versehen, der den Transport erleichtert. Gemäss einer in der Fig. 2 gezeigten bevorzugten Ausführungsform der vorliegenden Erfindung ist vorgesehen, dass der Schmelztiegel 1 direkt in den Wasserstoff- und Sauerstoffgenerator 10 integriert ist, der seinerseits mit einem Handgriff 11 versehen ist, der den Transport des Apparates erleichtert. Der Vorteil dieser Ausführungsform liegt offensichtlich darin, dass sich ein kompakter und stets betriebsbereiter Apparat ergibt, der sich ideal für die Zahnarztpraxis oder für den Operationssaal eignet.

Ein ähnlicher Apparat, gemäss einer bevorzugten Ausführungsform der vorliegenden Erfindung, weist kleine Abmessungen in Länge/Breite/Höhe von weniger als 45/45/35 cm auf, bei einem Gewicht von weniger als 40 kg und vorzugsweise weniger als 25 kg, so dass leichte Transportierbarkeit des Apparates auch für Pflege-Hilfspersonal gewährleistet ist.

Gemäss einer weiteren bevorzugten Ausführungsform der vorliegenden Erfindung, wie sie in den Figuren 1 und 2 dargestellt ist, weist der Schmelztiegel unterhalb des in den Figuren 1, 2 und 3 mit A bezeichneten Punktes, wo der Schmelzprozess stattfindet, einen herausziehbaren Behälter 12 auf, in welchem sich die bei der Verbrennung anfallenden Schlacken ansammeln. Der Behälter 12 seinerseits ist mit einem Handgriff 14 versehen, der bequemes Herausziehen aus dem Schmelztiegel 1 und einfache Leerung erlaubt.

Das Verfahren sieht sodann gemäss einer bevorzugten Realisierungsform vor, dass das Schmelzen des Instrumentes 5 ab einem Anfangspunkt fortschreitend bis zu einem Endpunkt erfolgt, wofür das Instrument 5 längs einer Bahn zwischen den beiden genannten Punkten fortschreitend gegen die Flamme 8 hin vorgeschoben wird. Offensichtlich kann dank diesem Vorgehen die vollständige Verbrennung auch von Instrumenten beträchtlicher Längenausdehnung, wie etwa Injektionsnadeln 6 von Spritzen oder Klingen von Messerchen 7, bewerkstelligt werden, obschon die verwendete Flamme nur kleine Abmessungen, aber lokal hohe thermische Leistung aufweist, also an einem genau festgelegten Punkt sehr heiss ist. Dies erlaubt die Verwendung von Apparaten zur Erzeugung der Flamme 8 von beschränkter Leistung (beispielsweise gemäss einer bevorzugten Realisierungsform der vorliegenden Erfindung zwischen 40 und 160 l/h Gasgemisch) sogar für das Vernichten von Instrumenten beträchtlicher Abmessungen.

Zu diesem Zweck ist entsprechend einer anderen Realisierungsform der vorliegenden Erfindung, erfindungsgemäss vorgesehen, dass der Schmelztiegel 1 bei der Einführungsöffnung 4 eine Führungsvorrichtung 15 aufweist, wie sie in der Fig. 2 dargestellt ist, die ein fortschreitendes Vorschieben des zu schmelzenden Instrumentes 5 zwischen einem Anfangspunkt und einem Endpunkt des Schmelzprozesses (also in der Regel zwischen der Spitze des Instrumentes und beispielsweise dem Punkt der Halterung der Nadel in der Spritze 6 und dem Anfang des eigentlichen Spritzenkörpers).

In der Fig. 3 ist die Führungsvorrichtung 15 im Sinne eines Beispiels als eine für den Transport nach unten in eine Ruhelage abklappbare Führungsebene dargestellt. Offensichtlich können jedoch auch andere Formen von Führungsvorrichtungen 15 ohne weiteres für die Ausbildung der Apparatur in Betracht gezogen werden (beispielsweise ein an die Öffnung 4 angeschlossener Führungstisch zum Einführen des Instrumentes 5 oder ein geschlossenes Einführungsröhrchen, das fest oder abnehmbar an der Öffnung 4 angebracht ist, usw.)

Die Art der Integration des Schmelztiegels 1 in den in der Fig. 2 gezeigten Wasserstoff- und Sauerstoffgenerator 10 stellt selbstverständlich bloss eine von vielen für diesen Zweck denkbaren Lösungen dar, auch weil die Abmessungen des hier gezeigten Schmelztiegels nicht unbedingt der Wirklichkeit entsprechen. So kann der Schmelztiegel im Verhältnis zum Generator 10 kleinere Abmessungen aufweisen als hier dargestellt, und er kann auch an einem anderen Platz innerhalb des Generators 10 untergebracht sein.

Der Vorteil des Verfahrens und der Vorrichtung in den beschriebenen Realisierungsformen besteht hauptsächlich darin, dass die schnelle und vollständige Vernichtung infizierter Instrumente möglich wird, wobei jede Infektionsgefahr durch allenfalls wegen fehlerhaftem Funktionieren unvollständig vernichtete Instrumententeile vermieden ist, eine Infektionsgefahr die in Vorrichtungen, die mit zwei Elektroden gemäss dem Stand der Technik arbeiten, stets vorhanden ist. Die erfindungsgemässe Vorrichtung erfordert bloss minimalen Unterhalt, der sich praktisch auf das Leeren der Schlacken beschränkt, die sich im dafür angeordneten Behälter 12 ansammeln.

### Liste der in den Figuren verwendeten Bezugsziffern

- 1: Schmelztiegel
- 2: Haube
- 3: Abzugsöffnung für Rauch
- 4: Einführungsöffnung für das Instrument
- 5: zu schmelzendes Instrument
- 6: Injektionsspritze
- 7: Messerchen
- 8: Flamme
- 9: Speiseröhrchen
- 10: Wasserstoff- und Sauerstoffgenerator
- 11: Handgriff
- 12: Behälter
- 13: Schlacke
- 14: Haltegriff
- 15: Führungsvorrichtung

## Patentansprüche

1. Verfahren zum Vernichten von chirurgischen Instrumenten durch Schmelzen, insbesondere von Injektionsnadeln, Klingen von Messerchen und Skalpellen,
**dadurch gekennzeichnet, dass**
der zu schmelzende Teil des Instrumentes (5) in direkten Kontakt mit einer Flamme (8) von höchster Temperatur gebracht wird, die durch Verbrennen eines Gemisches von Wasserstoff und Sauerstoff erzeugt wird.

2. Verfahren gemäss dem Anspruch 1,
**dadurch gekennzeichnet, dass**
die Temperatur der Flamme (8) höher als 3000°C ist.

3. Verfahren gemäss dem Anspruch 1,
**dadurch gekennzeichnet, dass**
das Schmelzen des Instrumentes (5) von einem Anfangspunkt aus fortschreitend bis zu einem Endpunkt erfolgt, wobei das Instrument (5) längs einer Bahn zwischen den beiden genannten Punkten fortschreitend gegen die Flamme (8) hin vorgeschoben wird.

4. Vorrichtung zum Ausüben des Verfahrens gemäss dem Anspruch 1,
**gekennzeichnet durch** die folgenden Konstruktionselemente:
- ein Schmelztiegel (1) mit einer Öffnung (4) zum Einführen des zu schmelzenden Instrumentes (6, 7),
- ein Speiseröhrchen (9) zum Einführen des Gasgemisches von Wasserstoff und Sauerstoff, das auf den Punkt im Innern des Schmelztiegels (1) ausgerichtet ist, an welchem das Instrument (5) geschmolzen wird,
- Sowie **durch** eine Abzugsöffnung (3) für den Rauch, der bei der Verbrennung des Gasgemisches entsteht,
- Ein Wasserstoff- und Sauerstoffgenerator (10), der auf elektrolytischer Dissoziation von Wasser basiert und der das Gasgemisch von Wasserstoff und Sauerstoff **durch** das Speiseröhrchen (9) einspeist.

5. Vorrichtung gemäss dem Anspruch 4,
**dadurch gekennzeichnet, dass**
der Schmelztiegel (1) direkt im Wasserstoff- und Sauerstoffgenerator (10) integriert ist.

6. Vorrichtung gemäss dem Anspruch 4,
**dadurch gekennzeichnet, dass**
der Schmelztiegel (1) unter dem Punkt (A), an welchem das Schmelzen erfolgt, einen herausziehbaren Behälter (12) aufweist, in welchem sich die beim Verbrennen anfallenden Schlacken (13) ansammeln.

7. Vorrichtung gemäss dem Anspruch 4,
**dadurch gekennzeichnet, dass**
der Schmelztiegel (1) ferner bei der Einführungsöffnung (4) eine Führungsvorrichtung (15) aufweist, mittels welcher das zu schmelzende Instrument (5) von einem Anfangspunkt an fortschreitend bis zu einem Endpunkt des Schmelzprozesses vorgeschoben wird.

8. Vorrichtung gemäss dem Anspruch 4,
**dadurch gekennzeichnet, dass**
der Wasserstoff- und Sauerstoffgenerator (10) mit einer Produktionsleistung von 40 bis 160 I/h arbeitet.

9. Vorrichtung gemäss dem Anspruch 4,
**dadurch gekennzeichnet, dass**
die Abmessungen der Vorrichtung in Länge/Breite/Höhe kleiner sind als 45/45/35 cm, und dass das Gewicht der Vorrichtung weniger als 40 kg und vorzugsweise weniger als 25 kg beträgt.

## Claims

1. Method for the destruction of surgical instruments by melting, in particular of injection needles, blades of small knifes and scalpels,
**characterised in that**
the part of the instrument (5) to be melted is brought into direct contact with a flame (8) of the highest temperature, which is produced by burning a mixture of hydrogen and oxygen.

2. Method in accordance with claim 1,
**characterised in that**
the temperature of the flame (8) is higher than 3000°C.

3. Method in accordance with claim 1,
**characterised in that**
the melting of the instrument (5) takes place progressively from a starting point up to an end point, with the instrument (5) being progressively advanced towards the flame (8) along a path between the two named points.

4. Apparatus for carrying out the method in accordance with claim 1, **characterised by** the following construction elements:
- a melting crucible (1) having an opening (4) for the introduction of the instrument (6, 7) to be melted,
- a feed tubule (9) for the introduction of the gas mixture of hydrogen and oxygen which is directed to the point in the interior of the melting crucible (1) at which the instrument (5) is melted,
- as well as an extraction opening (3) for the smoke which arises during the combustion of the gas mixture,
- a hydrogen and oxygen generator (10) which is based on the electrolytic dissociation of water and which feeds the gas mixture of hydrogen and oxygen through the feed tubule (9).

5. Apparatus in accordance with claim 4,
**characterised in that**
the melting crucible (1) is directly integrated into the hydrogen and oxygen generator (10).

6. Apparatus in accordance with claim 4,
**characterised in that**
the melting crucible (1) has an extractable container (12) below the point (A) at which the melting takes place and in which the slack (13) collects which occurs during burning.

7. Apparatus in accordance with claim 4,
**characterised in that**
the melting crucible (1) furthermore has a guide device (15) at the introduction opening (4) by means of which the instrument (5) to be melted is progressively advanced from a starting point up to an end point of the melting process.

8. Apparatus in accordance with claim 4,
**characterised in that**
the hydrogen and oxygen generator (10) operates with a production performance of 40 to 160 l/h.

9. Apparatus in accordance with claim 4,
**characterised in that**
the dimensions of the apparatus in length/width/height are smaller than 45/45/35 cm, and that the weight of the apparatus amounts to less than 40 kg and preferably to less than 25 kg.

## Revendications

1. Procédé de destruction d'instruments chirurgicaux par fusion, notamment d'aiguilles d'injection, de lames, de petits couteaux et de scalpels,
**caractérisé en ce que**
on met la partie à faire fondre de l'instrument (5) en contact direct avec une flamme (8) de température très haute qui est produite par combustion d'un mélange d'hydrogène et d'oxygène.

2. Procédé suivant la revendication 1,
**caractérisé en ce que**
la température de la flamme (8) est plus haute que 3000°C.

3. Procédé suivant la revendication 1,
**caractérisé en ce que**
on effectue la fusion de l'instrument (5) d'un point initial progressivement jusqu'à un point final en faisant avancer l'instrument (5) progressivement vers la flamme (8) le long d'un trajet entre les deux points mentionnés.

4. Dispositif pour effectuer le procédé suivant la revendication 1,
**caractérisé par** les éléments de construction suivants :
- un creuset (1) de fusion ayant une ouverture (4) d'introduction de l'instrument (6, 7) à faire fondre,
- un petit tube (9) d'alimentation pour introduire le mélange gazeux d'hydrogène et d'oxygène, le petit tube étant dirigé sur le point à l'intérieur du creuset (1) de fusion où l'instrument (5) est fondu,
- ainsi qu'une ouverture (3) d'évacuation de la fumée qui se produit lors de la combustion du mélange gazeux,
- un générateur (10) d'hydrogène et d'oxygène, qui repose sur la dissociation électrolytique de l'eau et qui injecte le mélange gazeux d'hydrogène et d'oxygène dans le petit tube (9) d'alimentation.

5. Dispositif suivant la revendication 4,
**caractérisé en ce que**
le creuset (1) de fusion est intégré directement au générateur (10) d'hydrogène et d'oxygène.

6. Dispositif suivant la revendication 4,
**caractérisé en ce que**
le creuset (1) de fusion a au point (A) où s'effectue la fusion un récipient (12) qui peut être retiré et dans lequel s'accumulent les scories (13) se produisant pendant la combustion.

7. Dispositif suivant la revendication 4,
**caractérisé en ce que**
le creuset (1) de fusion a, en outre, à l'ouverture (4) d'introduction un dispositif (15) de guidage, au moyen duquel l'instrument (5) à faire fondre est avancé du point initial progressivement jusqu'au point final du processus de fusion.

8. Dispositif suivant la revendication 4,
**caractérisé en ce que**
le générateur (10) d'hydrogène et d'oxygène fonctionne à un débit de production de 40 à 160 l/h.

9. Dispositif suivant la revendication 4,
**caractérisé en ce que**
les dimensions du dispositif en longueur/largeur/hauteur sont plus petites que 45/45/35 cm et **en ce que** le poids du dispositif est plus petit que 40 kg et, de préférence, plus petit que 25 kg.
